# EUROPEAN PATENT APPLICATION

(11) **EP 4 718 348 A1**
(43) Date of publication of application: **01.04.2026**
(21) Application number: 25205498.6
(22) Date of filing: 29.09.2025
(51) Int. Cl.: G06Q 10/04, G16H 50/30

(54) **PREDICTING AND MITIGATING EFFECTS OF ENVIRONMENTAL CONDITIONS**

(30) Priority: 30.09.2024 US 202418902253
(71) Applicant: Disney Enterprises, Inc., Burbank, CA 91521 (US)
(72) Inventor: Hale, Gregory Brooks, Orlando (GB); Markowitz, Gary David, San Juan Capistrano (US); Gilmore, David Benson, Orlando (US)
(74) Representative: Jarrett, Daniel Phillip

(57) **Abstract**

A system for predicting and mitigating an effect of environmental conditions, the system includes: a user device including a processor and a memory configured to: determine the environmental conditions for a location within an environment; analyze the environmental conditions in light of individual-specific criterion to determine a predicted impact on an individual within the location during the determined environmental conditions; and generate a mitigation response based on the predicted impact.

## Description

### FIELD

The present disclosure relates generally to systems and methods for mitigating the effects of environmental conditions.

### BACKGROUND

Exposure to environmental conditions can have deleterious effects on people, plants animals, and equipment. For example, people or animals exposed to heat and humidity, particularly while engaging in an activity (e.g., walking, running, etc.), can develop heat stress or other biological conditions. Heat stress or exhaustion typically generates physical symptoms such as sweating, weakness, dizziness, fainting, nausea, muscle cramps, headache, rapid heartbeat, etc. Similarly, plants may wilt, have stunted growth, be susceptible to disease or even die. Equipment may overheat or malfunction.

Current systems and methods to manage environmental exposures are deficient in numerous ways. For example, current methods may include issuing general heat advisories for a large area and for a population at large, in a one-size fits all approach. Further, existing systems also warn of the effects of exposure environmental conditions after the fact, when it is too late to avoid the effects.

### SUMMARY

Aspects and features of the present disclosure are set out in the appended claims.

### OVERVIEW OF DISCLOSURE

In one embodiment, a method of predicting an environmental exposure effect includes: receiving, via a processor, environmental data of an environmental condition; predicting, via the processor, a biological effect of the environmental condition on a user; and generating a notification regarding the predicted biological effect.

Optionally, in some embodiments, the method further includes receiving, via the processor, an activity plan of a user exposed to the environmental condition.

Optionally, in some embodiments, the method further includes adjusting, via the processor, the activity plan to avoid the biological effect.

Optionally, in some embodiments, the prediction is based on at least one of a biological characteristic of the user and/or an activity schedule of the user.

Optionally, in some embodiments, the processor is onboard a mobile device.

Optionally, in some embodiments, the environmental data includes at least one of a current environmental condition or a prediction of a future environmental condition.

Optionally, in some embodiments, the method further includes receiving, via the processor, a user tolerance to the environmental condition.

Optionally, in some embodiments, the method further includes generating, via the processor, a user alert indicating an imminent effect of exposure to the environmental condition.

Optionally, in some embodiments, the method further includes transmitting, via the processor, an adjusted activity plan to a user device; and displaying the adjusted activity plan on a display of the user device.

Optionally, in some embodiments, the method further includes receiving, via the processor, user health data from a user device.

Optionally, in some embodiments, the user device includes a wearable device.

Optionally, in some embodiments, the processor is associated with a user device and activity plan is stored on a memory of the user device.

Optionally, in some embodiments, the environmental data includes one or more of: a temperature, a heat exposure index, an insolation, a wind speed, a wind direction, a cloud cover, an atmospheric pressure, a precipitation amount, a wind chill, a dewpoint, a humidity, an atmospheric electrical field, a wind shear, an accumulated irradiation, or a level of a pollutant.

Optionally, in some embodiments, the pollutant is one or more of: an amount of a particulate matter, a nitrogen and oxygen compound, a sulfur compound, ozone, a hydrocarbon, carbon dioxide, or carbon monoxide.

In one embodiment, a method of predicting environmental exposure impact includes: determining environmental conditions for a location within an environment; analyzing the environmental conditions in light of individual-specific criterion to determine a predicted impact on an individual within the location during the determined environmental conditions; and generating a mitigation response based on the predicted impact.

Optionally, in some embodiments, the method further includes outputting the mitigation response to a display associated with a user device, wherein the user device is accessible by the individual.

Optionally, in some embodiments, the individual is one of a person, an animal, or a plant.

Optionally, in some embodiments, the individual-specific criterion includes an activity or route through the location.

Optionally, in some embodiments, the individual-specific criterion includes a biological characteristic associated with the individual.

In one embodiment, a system for predicting and mitigating an effect of environmental conditions, the system includes: a user device including a processor and a memory configured to: determine the environmental conditions for a location within an environment; analyze the environmental conditions in light of individual-specific criterion to determine a predicted impact on an individual within the location during the determined environmental conditions; and generate a mitigation response based on the predicted impact.

Optionally, in some embodiments, the processor and the memory are further configured to output the mitigation response to a display associated with the user device, wherein the user device is accessible by the individual.

Optionally, in some embodiments, the individual is one of a person, an animal, or a plant.

Optionally, in some embodiments, the individual-specific criterion includes an activity or route through the location.

Optionally, in some embodiments, the individual-specific criterion includes a biological characteristic associated with the individual.

### BRIEF DESCRIPTION OF THE DRAWINGS

FIG. 1 illustrates a simplified schematic of a system for predicting and mitigating the effects of environmental conditions according to embodiments herein.
FIG. 2 illustrates an example of a method performed by the system of FIG.1 for predicting and mitigating the effects of environmental conditions according to embodiments herein.
FIG. 3 illustrates an example of the method of FIG. 2 where the system may analyze the environmental data and, optionally, one or more user characteristics, according to embodiments herein.
FIG. 4A illustrates an example of the method of FIG. 2 to generate remediation and/or mitigation options.
FIG. 4B illustrates an example of the method of FIG. 2 to generate remediation and/or mitigation options.
FIG. 5 illustrates an example of the method of FIG. 2 where the system may provide a notification to the user of an upcoming exposure threshold according to embodiments herein.
FIG. 6 illustrates a simplified block diagram of components of a computing system of the system for predicting and mitigating the effects of environmental conditions of FIG. 1 according to embodiments herein.

### DETAILED DESCRIPTION

Embodiments disclosed herein predict and enable options to mitigate environmental exposure for people, animals, equipment, infrastructure, and/or plants. The prediction and mitigation may be done on an individual basis, allowing increased accuracy and responsiveness. Examples of environmental conditions used for prediction include temperature, humidity, ultraviolet (UV) exposure, air pollution or contamination (e.g., smoke, allergens), wind, altitude, ozone exposure, wind speed, current or wave height for water activities, topography, cloud cover, precipitation, elevation, season of the year (e.g., summer, winter, dry season, or monsoon), etc. The environmental conditions are analyzed based on a person (or other individual's) actual or predicted location (e.g., via location detection, user input, etc.). The method is configured to analyze various types of data from changing or non-static environments, typically those that are outdoors, including both land and water environments. The data is used, along with optional user or individual-specific data, including activity itineraries (e.g., work schedules, travel information, workout plan, race trajectory, etc.) and/or biological information (e.g., heart rate, blood sugar, breathing rate, prior exposure, health history, or tolerance information, recent meals, and the like) to predict exposure thresholds, e.g., when heat stress is likely to begin, maximum exposure time, exhaustion time frame. In some cases, the optional user or individual-specific data may include additional user specific information for biological and/or mental thresholds such as tolerance to noise, tolerance to highly crowded areas, tolerance to flashing lights and/or tolerance to exposure to new stimulants in the environment. Predictions can be determined before an activity has commenced, such as to allow changes to a particular itinerary ahead of time (e.g., allow scheduling of additional breaks or indoor activities) and/or may be done in real time to enable notifications to prevent crossing into an undesirable biological state.

Further, in some instances the effects of exposures to environmental conditions can be reset or reduced, if the biological systems (e.g., a person's body) have a chance to recover from a particular impact. For example, resting or reducing a heart rate from a running activity in the shade with a fan or in an air-conditioned room can allow a person to continue running in the sun for longer or further than if the person had not had a break. Various examples take such varying impact into effect when both making predictions about a particular itinerary or schedule and when providing real time feedback.

The systems and methods disclosed predict environmental conditions, including the effects (particularly the cumulative effects) thereof. By predicting the effects of exposure to the environmental conditions, the systems can adjust an activity plan to help avoid or mitigate the impacts of those exposures. The systems can also generate notifications that an exposure limit is imminent to allow a person to take protective action ahead of experiencing an issue.

Various embodiments allow many activities to be continued in a more comfortable and less harmful manner, by helping to identify and prevent over exposure within a particular environment. This may allow construction work, athletes, tourists, and the like to more efficiently, enjoyably, and productively maintain a desired activity schedule, even in harsh environmental conditions, such as hot, humid summer days.

It should be noted that many examples are discussed with respect to people, but are equally applicable to other biological systems, including animals, vegetation, or the like that experience similar physiological issues when experiencing certain environmental conditions (e.g., plants that wither in the heat or the like). Similarly, there are certain foods, equipment, and non-living objects that may also have impact thresholds for which these embodiments could be used to predict and mitigate the impacts. Therefore, the discussion of any particular example is meant as illustrative only.

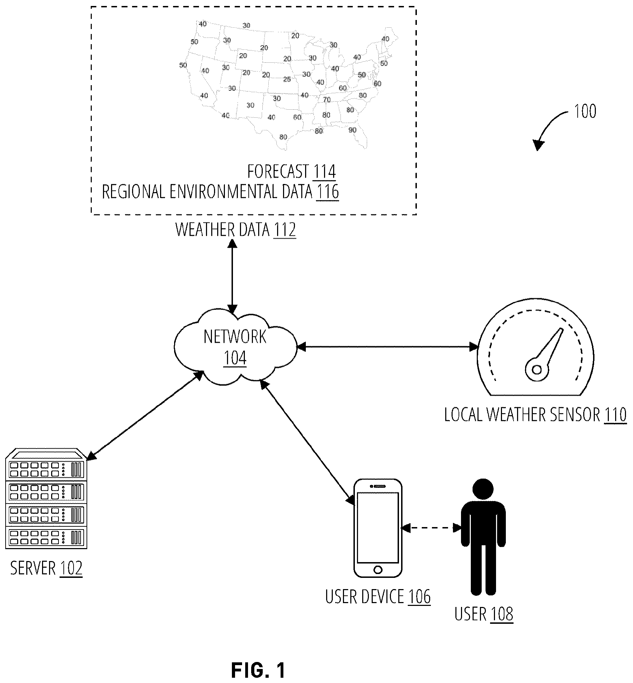

Turning to the figures, FIG. 1 illustrates a simplified schematic of a system 100 for predicting and optionally enabling mitigation of effects of environmental conditions. The system 100 analyzes environmental conditions to predict and mitigate the effects of prolonged exposure of an individual (e.g., user) to the various environmental conditions. The system 100 predicts exposure thresholds or time periods, e.g., when heat stress is likely to begin or when exhaustion or aerobic performance may dip. The predictions can be made before an activity has commenced, such as to allow changes to a particular itinerary ahead of time (e.g., allow scheduling of additional breaks or indoor activities) and/or may provide real time notifications to prevent crossing into an undesirable biological state while an activity is occurring (e.g., generate an alert that a rest may be beneficial to reduce heat stress).

The system 100 includes a server 102 and a user device 106 (e.g., a computing device that a user 108 may interact with such as a phone, tablet, smart watch, head-mounted display, or other wearable device, a laptop or desktop computer, etc. ), a network 104, and one more environmental characteristic databases or sensors, including as an example, a local environmental sensor 110 (e.g., temperature sensor, humidity sensor, etc.), weather data 112 including the forecast 114 and regional environmental data 116.

In some instances, the network 104 receives environmental conditions or characteristics, such as weather data 112 of a location where the user 108 will be or is performing activities or is residing at including current and/or future weather information such as the forecast 114 and regional environmental data 116. The weather data 112 may include, for example, the current temperature, the predicted future temperature and how the temperature changes throughout the day, current and/or predicted future precipitation, current and/or predicted future ultraviolent (UV) index, humidity, pressure, an insolation, a wind speed, a wind direction, a cloud cover, an atmospheric pressure, a precipitation amount, a wind chill, a dewpoint, a humidity, an atmospheric electrical field, a wind shear, an accumulated irradiation, a level of a pollutant, air quality, a smoke index, altitude, air pollution or contamination (e.g., an amount of a particulate matter, a nitrogen and oxygen compound, a sulfur compound, ozone, a hydrocarbon, carbon dioxide, or carbon monoxide). It should be understood that this is not an exhaustive list and the weather data 112 may include various types of current and/or predicted future pertinent environmental data.

Additionally, the network 104 may receive local environmental conditions from a local weather sensor 110. The local environmental conditions received from the local weather sensor 110 may be more detailed for a specific location as compared to weather data 112 that may apply to a more generalized area. For example, it may be that the location that the user 108 will be performing activities at or is residing at (e.g., work site, outdoor sport complex, garden, outdoor shopping center) has its own local barometer, anemometer thermometer, hygrometer, rain gauge, radar, or weather instrument of the sort that may provide detailed environmental conditions or characteristics for the specific location where the user 108 is located.

Optionally, in some examples, the user device 106 may include user or individual-specific data such as activity itineraries (e.g., work schedules, travel information, etc.) and/or biological information (e.g., heart rate, breathing rate, prior exposure or tolerance information, health history and the like). In some instances, the information user or individual-specific data may be received from, for example, on board sensors, a third party database (e.g., work calendars, travel agents, doctors), and/or from input received from the user 108. The optional user or individual-specific data may be used by the system 100 for predicting and mitigating the effect of the environmental conditions and or characteristics.

The system 100 may make predictions regarding exposure thresholds (e.g., when heat stress is likely to begin) based on the received weather data 112, the local environmental conditions from the local weather sensor 110 and/or the optional user or individual-specific data. The system may perform such predictions on board of the user device 106 or off board of the user device.

In some cases, the system 100 may also generate remedial and/or mitigation options to help ensure the user does not cross the exposure threshold. The generated remedial and/or mitigation options may help reset the biological consequences incurred with exposure the environmental conditions and/or characteristics, reducing the effects of the environmental conditions and/or characteristics. For example, the remedial and/or mitigation options may take the form of an alert based on the prediction or may take the form of a plan to help extend the exposure time (e.g., sit under a tree for 10 minutes when approaching a sun exposure threshold reducing biological exertion or heart rate, or the like).

The system 100 may output the prediction of the approaching exposure threshold and, in some instances, the generated remedial and/or mitigating options to the user 108 via the user device 106. For example, the user device 106 may receive from the system 100 (e.g., from a device via the network 104), information that the user is predicted to cross a sun/heat exposure threshold after a certain amount of time exposed to the sun/heat and notify the user 108. In some instances, the user 108 may be recommended via the user device 106 to find shade as to get out of the sun or to put on sun/heat protective gear. In another example, the user 108 may be notified via the user device 106 to move to a quieter location or put on hearing protection as the user 108 may approach a noise exposure threshold after a certain amount of time exposed. In yet another example, the user 108 may be provided via the user device 106 with optional itinerary changes/updates ahead of time (e.g., allow scheduling of additional breaks or indoor activities) as to not approach the predicted exposure thresholds that the user may encounter if the original itinerary is performed without changes/updates. In some instances, it may be that a portion of the optional itinerary changes are approved/accepted by the user 108 and a second portion of the optional itinerary changes are declined by the user 108.

The system 100 may also transmit the prediction regarding the exposure thresholds to the server 102 for storage. Similarity, the system 100 may transmit the remedial and/or mitigation options (if generated) to the server 102 for storage. In some instances, previously stored prediction may be forwarded to the network 104 or user device 106 for use in future exposure threshold predictions as to predict a more accurate timing of when the exposure threshold may be crossed by the user 108. Similarly, the stored mitigation options may be forwarded to the network 104 or the user device 106 for use in future mitigation option generation. As a result, the prediction of the approaching exposure threshold and generated mitigation options may be optimized based on previous predictions and generated mitigation options.

Additionally, in some cases, the prediction and the mitigation options stored at the server 102 may be used to include and/or remove features of the environment. For example, if the prediction and mitigation options stored at the server 102 indicate that many predictions have been made by the system 100 that users 108 tend to approach a heat exposure threshold at a certain location and/or environment, it may be beneficial to add mitigating features (e.g., shade, drinking water fountains, misting system, benches, an indoor enclosure, rest areas)as to prevent future users 108 from approaching the heat exposure threshold in said location.

Although the preceding examples detail a user 108 interacting with a user device 106, the disclosure is also applicable to, for example, plants, animals, transportation infrastructure, and any other entities that may be exposed to environmental conditions and characteristics and may incur biological consequences as a result of crossing an exposure threshold. The various functions may be done by one or more of the devices within the system, e.g., the user device 106 or a server 102 may receive the user information and the weather information to make predictions and the description of any particular device performing a particular operation is meant as illustrative only.

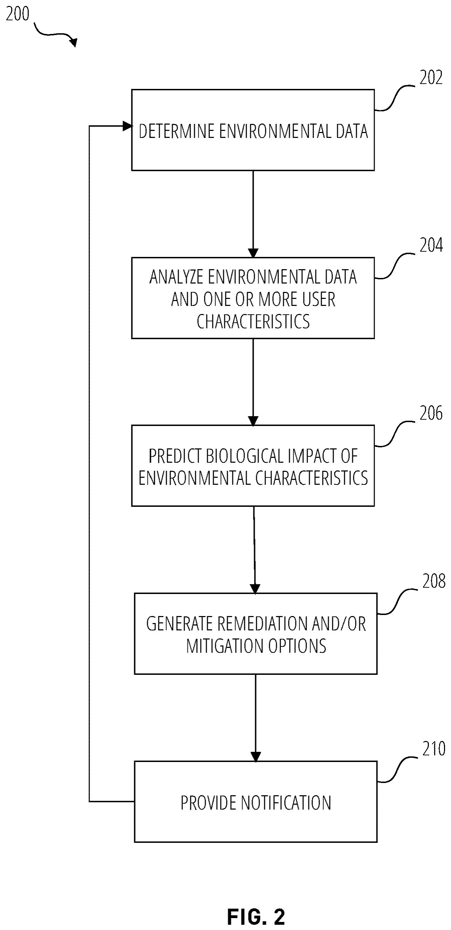

FIG. 2 illustrates an example of a method 200 performed by the system 100 for predicting and mitigating the effects of environmental conditions according to embodiments herein. Although the example method 200 depicts a particular sequence of operations, the sequence may be altered without departing from the scope of the present disclosure. For example, some of the operations depicted may be performed in parallel or in a different sequence that does not materially affect the function of the method 200. In some examples, the method 200 may be executed by a single device, while in other examples, the method 200 may be distributed across different devices (e.g., server 102, the user device 106, and/or the network 104) with each device performing a part or all of the method 200. In some examples, the method 200 may be executed by different components of an example device of the system 100 that implements the method 200. In some examples, the operations of the method 200 may be performed at substantially the same time or in a specific sequence.

The method 200 may begin by the system 100 determining environmental data at operation 202. For example, in operation 202 the network 104 may receive weather data 112 including and/or local weather information from a local weather sensor 110 to determine environmental data and/or environmental condition of a location where the user will be residing or performing activities.

The method 200 may proceed to operation 204 where the system 100 may analyze the environmental data and, optionally, one or more user characteristics. For example, in operation 204 the network 104 may analyze the weather data 112, local weather information from the local weather sensor 110 and optionally user or individual-specific information to determine environmental conditions and/or characteristics that the user 108 may encounter.

The method 200 may proceed to operation 206 where the system 100 may predict one or more biological impacts of the environmental conditions and/or characteristics on the user 108. For example, in operation 206 the system 100 may predict exposure thresholds for the user 108 based on the environmental conditions and/or characteristics that when crossed may incur biological consequences for the user 108 such as heat stroke when a heat exposure threshold is encountered.

The method 200 may proceed to operation 208 where the system 100 may generate remediation and/or mitigation options to avoid crossing an exposure threshold corresponding to the biological impact of the environmental characteristics. For example, in operation 208 the system 100 may generate remediation and/or mitigation options to avoid crossing the predicted exposure threshold. In some cases, the remediation and/or mitigation options may take the form of recommending to the user to put on sun protection or finding an area with shade when approaching a sun and/or heat exposure threshold, putting on sound reduction head phones when approaching a noise exposure threshold, changing the order of an itinerary to occur earlier in the day or reordering events of the itinerary if the system 100 determines that the original itinerary if performed may approach any exposure thresholds.

The method 200 may proceed to operation 210 where the system 100 may provide a notification to the user 108 of the approaching exposure threshold. In some cases, the system 100 may also notify the user 108 via the user device 106 of the generated remediation and/or mitigation options to avoid crossing the upcoming exposure threshold. For example, in operation 210 the system 100 may notify the user 108 via the user device 106 of the upcoming exposure threshold crossing and, in some cases, possible remediation and/or mitigation options. In certain examples, the user 108 may decline the optional remediation and/or mitigation options. In some such examples, the system 100 may occasionally notify the user 108 of the approaching exposure threshold even if the user 108 has declined the optional remediation and/or mitigation steps. In certain other examples, the user 108 may be notified of only the approaching exposure threshold with no remediation and/or mitigation steps recommended. In other examples, the predictions may be provided to the user device or the data to generate the predictions and the user device, such as via a health or activity application on the device, may be used to generate alerts and notifications, as well as recommended actions. In these instances, the user device may also include historical performance (e.g., biological information, heart rate, etc.) that can be used to create more accurate user specific predictions and recommendations.

In some instances, the method 200 may proceed back to operation 202 where the system 100 may repeat the method 200 by again determining environmental data of the location of the user. For example, the system 100 may continuously perform method 200 as to notify the user 108 of any upcoming exposure thresholds as the environmental conditions may change throughout the day (e.g., temperature change, humidity change, UV index change, air pollution change). In some other examples, the biological effects of the exposure may be reset as the user 108 may have taken one or more of the various remediation or mitigation options or performed their own remediation or mitigation options not provided by the system 100 and the system 100 may once again predict if the user 108 is approaching or will approach any exposure thresholds.

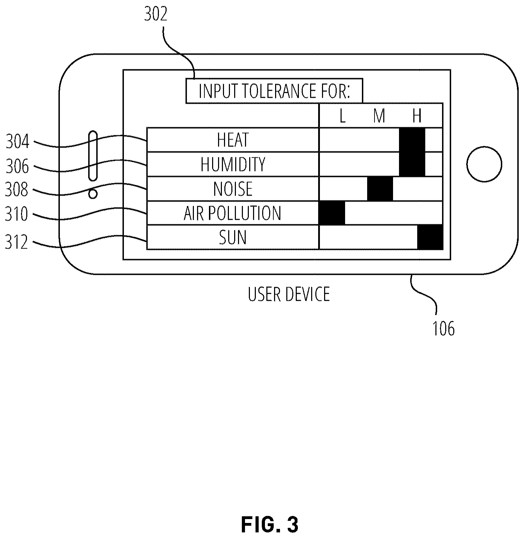

FIG. 3 illustrates an example of the method 200 where the system 100 may analyze the environmental data and, optionally, one or more user characteristics, according to embodiments herein.

In some instances, of the method 200, the system 100 may analyze environmental data and optionally, user or individual-specific information on the user device 106. For example, the user device 106 may include a user tolerance 302 to various environmental conditions and/or characteristics received from, for example, on board sensors of the user device 106, a third party database (e.g., work calendars, travel agents, doctors), and/or from input received from the user 108. It should be understood that the user 108 may indicate that they do not want to provide any user or individual-specific information.

As illustrated in FIG. 3, optionally the system 100 may be provided with user or individual-specific information encompassing the users' tolerances to various environmental conditions and/or characteristics to be used in the exposure threshold prediction. For example, the system 100 may be provided with user or individual-specific information indicating that the user may have a high tolerance to heat exposure 304, a high tolerance to humidity exposure 306, a medium tolerance to noise, a low tolerance to air pollution exposure 310 and a high tolerance to sun exposure 312. The system 100 may predict, based in part on the tolerances, that user 108 may approach an air pollution exposure 310 threshold sooner compared to a heat exposure 304, a humidity exposure 306, a noise exposure 308 and a sun exposure 312 threshold if exposed to such environmental conditions. Additionally, in some cases the system 100 may generate and provide remediation and/or mitigation options to reduce air pollution exposure 310 as the system 100 has been provided with user or individual-specific information indicating that the user 108 may have a low tolerance to air pollution exposure 310.

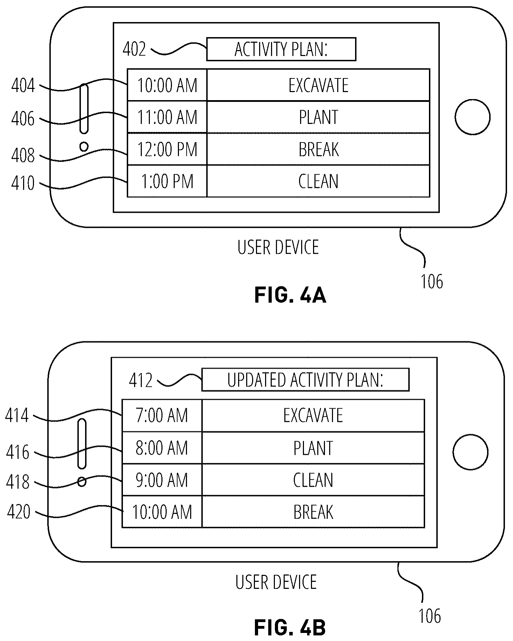

FIG. 4A illustrates an example of the method of FIG. 2 to generate remediation and/or mitigation options.

Consider an example where a user 108 is planting flowers in a garden. The system 100 may be provided with an initial activity plan 402 set in the user device 106 that the user 108 may perform including excavating 404 at 10 AM, planting 406 at 11 AM, taking a break 408 at 12 PM and cleaning 410 at 1 PM. According to embodiments herein, the system 100 may predict that the user 108 may cross a sun exposure threshold and a heat exposure threshold if the user 108 performs the activity plan 402 in its current form as the user may be excavating 404, planting 406 and cleaning 410 when sun exposure is high and the user 108 may be exposed to high/increasing temperatures.

FIG. 4B illustrates an example of the method of FIG. 2 to generate remediation and/or mitigation options. In some embodiments, the system 100 may generate an updated activity plan 412 that mitigates the heat/sun exposure. The system 100 may notify the user 108 via the user device 106 that if the original activity plan 402 is performed there may be approaching exposure thresholds and, in some instances, may provide the user 108 via the user device 106 with the updated activity plan 412. Note that the user 108 may optionally accept and perform (or accept only parts of) the updated activity plan 412. The updated activity plan 412 may include excavating 414 at 7AM, planting 416 at 8 AM, cleaning 418 at 9AM and taking a break 420 at 10 AM. As a result, the updated activity plan 412 may limit the user's exposure to the sun and heat as the updated activity plan 412 occurs earlier in the day with lower temperatures and lower sun exposure. The updated activity plan 412 may mitigate the chance of crossing a heat exposure threshold and/or sun exposure threshold and therefore the biological consequences of crossing the exposure thresholds.

In some instances, the user 108 may decline the updated activity plan 412 and may perform the activity plan 402 as initially set. In such instances, the user 108 may still be notified, while performing the activity plan 402, that a predicted exposure threshold (e.g., sun exposure and/or heat exposure) may be approached in the future and to take remediation and/or mitigation steps to avoid crossing the exposure threshold.

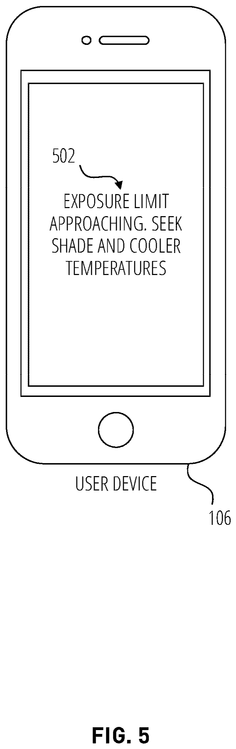

FIG. 5 illustrates an example of operation 210 of method 200 where the system 100 may provide a notification to the user 108 of an upcoming exposure threshold, according to embodiments herein.

In some embodiments, the system 100 may predict that the user 108 may approach an exposure threshold and, in response, may generate remediation and/or mitigation options to mitigate the approaching exposure threshold. The system 100 may also notify 502 the user of the predicted exposure threshold and of the generated remediation and/or mitigation options. For example, the user 108 may be notified 502 via the user device 106 that an "exposure limit is approaching" and "to seek shade and cooler temperatures." In some cases, the user may disregard the notification and decline performing the remediation and/or mitigation options.

As some specific examples of the method and system described herein, consider an example where a user is working outside at a job site. To begin, the user may optionally input into a user device the user's tolerances for various environmental conditions such as sun exposure, humidity, heat, etc. Then, embodiments herein may predict that the user may approach a heat exposure threshold at a certain time (e.g., 1 PM) due to the increased sun and heat exposure as predicted based on, for example, local environmental conditions, the forecast, and optional user data. Various mitigating options may be generated for the user as to not cross the heat exposure threshold. For example, it may be recommended for the user to reduce the intensity they are working at, to take a break, to drink some water, and/or to work in a shaded area of the job site. Then, the user may be notified of the approaching exposure threshold and of the mitigating options. In some cases, the user may perform the mitigating options or decline to perform the mitigating options but still be reminded of the approaching exposure threshold. Optionally, the predicted approaching exposure threshold and recommended mitigation options may be notified to the manager of the user or to a job site supervisor and the manager or the job site supervisor may recommend the user to take a break and schedule a second user for the job site while the first takes a break as to maintain a high level of work efficiency and output while also maintaining worker safety (i.e., preventing the user from incurring biological consequences due to crossing the exposure threshold).

Consider an example where a plant is growing in a location with an unstable climate under the supervision of a farmer. According to embodiments herein, it may be predicted based on local weather data showing an unexpected increase in sun and/or heat, that the plant may approach a heat exposure threshold in the afternoon and as a consequence may wilt and/or die. Mitigating options such as covering the plant or watering the plant may be generated. The farmer may be notified of the approaching heat exposure threshold of the plant and the generated mitigation options as to prevent the wilting of or death of the plant.

Consider an example where a user is attending an amusement park. Initially, the user may create or optionally may be provided with an itinerary including an order of rides and when to take food and/or rest breaks. According to embodiments herein, it may be predicted that the user may approach and even cross an air pollution exposure threshold if the initially set/provided itinerary is followed due to, for example, smoke from a nearby wildfire. The user may be notified of the possibility of crossing the air pollution exposure threshold and may be recommended an updated itinerary to mitigate the air pollution exposure threshold (e.g., an itinerary including taking breaks and enjoying indoor rides). The user may either accept or decline the updated itinerary (or accept/decline parts of the updated itinerary). Optionally, the user may provide user data such as tolerances to certain exposures as to be recommended a better fitting updated itinerary that prevents approaching any exposure thresholds.

Consider an example where frozen food is being transported in a semi-truck across the country. According to embodiments herein, it may be predicted that the frozen food being transported by the semi-truck may be at risk of melting as the frozen food may approach a melting threshold as the current navigation of the semi-truck may cause the semi-truck to be stuck in standstill traffic in a hot climate. Mitigating options may be generated for the semi-truck driver (or the dispatcher of the semi-truck driver) such as taking a route with less traffic, taking a navigation route with lower temperatures or more shade, or offloading the frozen food at a nearby facility and waiting for unfavorable conditions to pass. The semi-truck driver (or the dispatcher of the semi-truck driver) may be notified of the predicted approaching melting threshold and the recommended mitigating options. The semi-truck driver may perform one or more of the mitigation options or may decline the mitigation options while still being notified of the approaching melting threshold.

Consider an example where athletes are undergoing summer pre-season training. According to embodiments herein, it may be predicted that a certain group of athletes may approach a sun and/or heat exposure threshold as they have a lower tolerance to heat and sun exposure (as compared to other athletes). As a result, a coach, a medical supervisor, or the athlete themselves may be notified that the athletes will approach a sun/heat exposure threshold at a certain time in the day. It may be recommended, for example, for the athlete to reduce their training intensity as to train for the entirety of the training session, take a water break, or train in a climate controlled environment during high levels of environmental exposure. Beneficially, this may reduce any biological consequences (e.g., fainting and/or heat stress) that may occur when training while approaching the heat and sun exposure threshold.

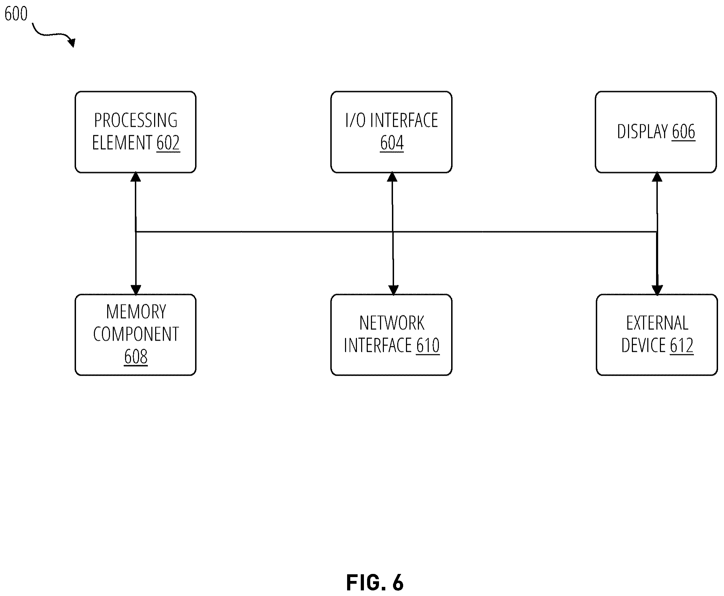

FIG. 6 is a simplified block diagram of components of a computing system 600 of the system 100, such as the server 102, a local weather sensor 110, the user device 106 etc. For example, the processing element 602 and the memory component 608 may be located at one or in several computing systems 600. This disclosure contemplates any suitable number of such computing systems 600. For example, the server 102 may be a desktop computing system, a mainframe, a blade, a mesh of computing systems 600, a laptop or notebook computing system 600, a tablet computing system 600, an embedded computing system 600, a system-on-chip, a single-board computing system 600, or a combination of two or more of these. Where appropriate, a computing system 600 may include one or more computing systems 600; be unitary or distributed; span multiple locations; span multiple machines; span multiple data centers; or reside in a cloud, which may include one or more cloud components in one or more networks. A computing system 600 may include one or more processing elements 602, an input/output I/O interface 604, one or more external devices 612, one or more memory components 608, and a network interface 610. Each of the various components may be in communication with one another through one or more buses or communication networks, such as wired or wireless networks, e.g., the network 104. The components in FIG. 6 are exemplary only. In various examples, the computing system 600 may include additional components and/or functionality not shown in FIG. 6.

The processing element 602 may be any type of electronic device capable of processing, receiving, and/or transmitting instructions. For example, the processing element 602 may be a central processing unit, microprocessor, processor, or microcontroller. Additionally, it should be noted that some components of the computing system 600 may be controlled by a first processing element 602 and other components may be controlled by a second processing element 602, where the first and second processing elements may or may not be in communication with each other.

The I/O interface 604 allows a user to enter data in to computing system 600, as well as provides an input/output for the computing system 600 to communicate with other devices or services. The I/O interface 604 can include one or more input buttons, touch pads, touch screens, and so on.

The external device 612 are one or more devices that can be used to provide various inputs to the computing systems 600, e.g., mouse, microphone, keyboard, trackpad, sensing element (e.g., a thermistor, humidity sensor, light detector, etc. The external devices 612 may be local or remote and may vary as desired. In some examples, the external devices 612 may also include one or more additional sensors.

The memory components 608 are used by the computing system 600 to store instructions for the processing element 602, as well as store data, such as weather data 112 including a forecast 114 and regional environmental data 116. The memory components 608 may be, for example, magneto-optical storage, read-only memory, random access memory, erasable programmable memory, flash memory, or a combination of one or more types of memory components.

The network interface 610 provides communication to and from the computing system 600 to other devices. The network interface 610 includes one or more communication protocols, such as, but not limited to Wi-Fi, Ethernet, Bluetooth, etc. The network interface 610 may also include one or more hardwired components, such as a Universal Serial Bus (USB) cable, or the like. The configuration of the network interface 610 depends on the types of communication desired and may be modified to communicate via Wi-Fi, Bluetooth, etc.

The display 606 provides a visual output for the computing system 600 and may be varied as needed based on the device. The display 606 may be configured to provide visual feedback to the user 108 and may include a liquid crystal display screen, light emitting diode screen, plasma screen, or the like. In some examples, the display 606 may be configured to act as an input element for the user 108 through touch feedback or the like.

Any description of a particular component being part of a particular embodiment, is meant as illustrative only and should not be interpreted as being required to be used with a particular embodiment or requiring other elements as shown in the depicted embodiment.

All relative and directional references (including top, bottom, side, front, rear, and so forth) are given by way of example to aid the reader's understanding of the examples described herein. They should not be read to be requirements or limitations, particularly as to the position, orientation, or use unless specifically set forth in the claims. Connection references (e.g., attached, coupled, connected, joined, and the like) are to be construed broadly and may include intermediate members between a connection of elements and relative movement between elements. As such, connection references do not necessarily infer that two elements are directly connected and in fixed relation to each other, unless specifically set forth in the claims.

The present disclosure teaches by way of example and not by limitation. Therefore, the matter contained in the above description or shown in the accompanying drawings should be interpreted as illustrative and not in a limiting sense. The following claims are intended to cover all generic and specific features described herein, as well as all statements of the scope of the present method and system, which, as a matter of language, might be said to fall there between.

Examples of the present disclosure are set out in the following numbered clauses.
1. A method of predicting an environmental exposure effect comprising:
   receiving, via a processor, environmental data of an environmental condition;
   predicting, via the processor, a biological effect of the environmental condition on a user; and
   generating a notification regarding the predicted biological effect.
2. The method of clause 1, further comprising receiving, via the processor, an activity plan of a user exposed to the environmental condition.
3. The method of clause 2, further comprising adjusting, via the processor, the activity plan to avoid the biological effect.
4. The method of clause 1, wherein the prediction is based on at least one of a biological characteristic of the user and/or an activity schedule of the user.
5. The method of clause 1, wherein the environmental data comprises at least one of a current environmental condition or a prediction of a future environmental condition.
6. The method of clause 1, further comprising:
   receiving, via the processor, a user tolerance to the environmental condition.
7. The method of clause 1, further comprising:
   generating, via the processor, a user alert indicating an imminent effect of exposure to the environmental condition.
8. The method of clause 1, further comprising:
   transmitting, via the processor, an adjusted activity plan to a user device; and
   displaying the adjusted activity plan on a display of the user device.
9. The method of clause 1, further comprising:
   receiving, via the processor, user health data from a user device.
10. The method of clause 9, wherein the processor is associated with a user device and activity plan is stored on a memory of the user device.
11. The method of clause 1, wherein the environmental data comprises one or more of:
   a temperature, a heat exposure index, an insolation, a wind speed, a wind direction, a cloud cover, an atmospheric pressure, a precipitation amount, a wind chill, a dewpoint, a humidity, an atmospheric electrical field, a wind shear, an accumulated irradiation, or a level of a pollutant.
12. The method of clause **11,** wherein the pollutant is one or more of: an amount of a particulate matter, a nitrogen and oxygen compound, a sulfur compound, ozone, a hydrocarbon, carbon dioxide, or carbon monoxide.
13. A method of predicting environmental exposure impact comprising:
   determining environmental conditions for a location within an environment;
   analyzing the environmental conditions in light of individual-specific criterion to determine a predicted impact on an individual within the location during the determined environmental conditions; and
   generating a mitigation response based on the predicted impact.
14. The method of clause 13, further comprising outputting the mitigation response to a display associated with a user device, wherein the user device is accessible by the individual.
15. The method of clause 13, wherein the individual is one of a person, an animal, or a plant.
16. The method of clause 13, wherein the individual-specific criterion includes an activity or route through the location.
17. A system for predicting and mitigating an effect of environmental conditions, the system comprising:
   a user device including a processor and a memory configured to:
   determine the environmental conditions for a location within an environment;
   analyze the environmental conditions in light of individual-specific criterion to determine a predicted impact on an individual within the location during the determined environmental conditions; and
   generate a mitigation response based on the predicted impact.
18. The system for of clause 17, wherein the processor and the memory are further configured to output the mitigation response to a display associated with the user device, wherein the user device is accessible by the individual.
19. The system for of clause 17, wherein the individual is one of a person, an animal, or a plant.
20. The system for of clause 17, wherein the individual-specific criterion includes a biological characteristic associated with the individual.

## Claims

1. A method of predicting an environmental exposure effect comprising:
receiving, via a processor, environmental data of an environmental condition;
predicting, via the processor, a biological effect of the environmental condition on a user; and
generating a notification regarding the predicted biological effect.

2. The method of claim 1, further comprising receiving, via the processor, an activity plan of a user exposed to the environmental condition.

3. The method of claim 2, further comprising adjusting, via the processor, the activity plan to avoid the biological effect.

4. The method of any preceding claim, wherein the predicting is based on at least one of a biological characteristic of the user and/or an activity schedule of the user.

5. The method of any preceding claim, wherein the environmental data comprises at least one of a current environmental condition or a prediction of a future environmental condition.

6. The method of any preceding claim, further comprising at least one of:
receiving, via the processor, a user tolerance to the environmental condition; or
generating, via the processor, a user alert indicating an imminent effect of exposure to the environmental condition.

7. The method of any preceding claim, further comprising:
transmitting, via the processor, an adjusted activity plan to a user device; and
displaying the adjusted activity plan on a display of the user device.

8. The method of any preceding claim, further comprising:
receiving, via the processor, user health data from a user device,
optionally wherein the processor is associated with a user device and activity plan is stored on a memory of the user device.

9. The method of any preceding claim, wherein the environmental data comprises one or more of:
a temperature, a heat exposure index, an insolation, a wind speed, a wind direction, a cloud cover, an atmospheric pressure, a precipitation amount, a wind chill, a dewpoint, a humidity, an atmospheric electrical field, a wind shear, an accumulated irradiation, or a level of a pollutant,
optionally wherein the pollutant is one or more of: an amount of a particulate matter, a nitrogen and oxygen compound, a sulfur compound, ozone, a hydrocarbon, carbon dioxide, or carbon monoxide.

10. A computer-implemented method of predicting environmental exposure impact comprising:
determining environmental conditions for a location within an environment;
analyzing the environmental conditions in light of individual-specific criterion to determine a predicted impact on an individual within the location during the determined environmental conditions; and
generating a mitigation response based on the predicted impact.

11. The method of claim 10, further comprising outputting the mitigation response to a display associated with a user device, wherein the user device is accessible by the individual.

12. The method of any of claims 10 to 11, wherein the individual is one of a person, an animal, or a plant.

13. The method of any of claims 10 to 12, wherein the individual-specific criterion includes at least one of:
an activity or route through the location; or
a biological characteristic associated with the individual.

14. A computing system configured to perform the method of any of claims 1 to 13.

15. A memory component storing instructions which, when executed by a processing element of a computing system, cause the computing system to perform the method of any of claims 1 to 13.
